# EUROPÄISCHE PATENTSCHRIFT

(11) **EP 3 164 183 B1**
(45) Veröffentlichungstag und Bekanntmachung des Hinweises auf die Patenterteilung: **09.05.2018**
(21) Anmeldenummer: 15734664.4
(22) Anmeldetag: 06.07.2015
(51) Int. Cl.: A61M 16/00, A62B 9/02, A61M 16/12, A61M 16/20, A61M 39/22, A61M 16/08

(54) **BEATMUNGSVORRICHTUNG**
RESPIRATORY DEVICE
RESPIRATEUR

(30) Priorität: 04.07.2014 DE 102014109394
(43) Veröffentlichungstag der Anmeldung: 10.05.2017
(73) Patentinhaber: Kagan, Eugen, 53859 Niederkassel Lülsdorf (DE)
(72) Erfinder: Kagan, Eugen, 53859 Niederkassel Lülsdorf (DE)
(74) Vertreter: Wagner Albiger & Partner Patentanwälte mbB
(86) Internationale Anmeldenummer: PCT/EP2015/065364
(87) Internationale Veröffentlichungsnummer: WO 2016/001448

(56) Entgegenhaltungen:
- EP-A1- 1 064 043
- EP-A2- 1 205 203
- US-A1- 2003 150 456
- US-A1- 2009 241 960
- US-A1- 2013 276 789
- US-B2- 8 047 205

## Beschreibung

### Stand der Technik

Die Erfindung geht von einer Beatmungsvorrichtung nach dem Oberbegriff des Anspruchs 1 aus.

Herkömmliche Beatmungsvorrichtungen werden dazu verwendet, einem Patienten eine geeignete Mischung aus Sauerstoff und Luft zuzuführen, um den Patienten zum Beispiel vollständig künstlich zu beatmen und/oder seine Atmung zumindest teilweise zu unterstützen. Es ist jedoch bei herkömmlichen Beatmungsvorrichtungen stets eine Druckluftversorgung erforderlich, die über eine Luftdruckflasche oder ein stationäres Druckluftversorgungssystem, zum Beispiel in einem Krankenhaus, zur Verfügung gestellt werden muss. Daher ist bei herkömmlichen Beatmungsvorrichtungen ein mobiler Einsatz, d.h. ohne Luftdruckflasche oder Druckluftversorgungssystem, nicht möglich, so dass die Einsatzmöglichkeiten herkömmlicher Beatmungsvorrichtungen auf den in diesem Sinne stationären Betrieb auch eingeschränkt sind. Im mobilen Einsatz gibt es sauerstoff- oder turbinengetriebene Geräte.
Aus der US 8,047 205 B2 und der EP 1 064 043 A1 sind Beatmungsvorrichtungen bekannt, die neben einer Druckluftversorgung auch eine Versorgung mit angesaugter Umgebungsluft erlauben. Allerdings weisen diese Beatmungsvorrichtungen Gasmischtanks zum Mischen geeignet zusammengesetzter Atemluft auf und sind daher für den mobilen Einsatz nur bedingt geeignet.

Es ist daher die Aufgabe der vorliegenden Erfindung, eine Beatmungsvorrichtung zur Verfügung zu stellen, die die Nachteile des Stands der Technik nicht aufweist und insbesondere einen Betrieb im stationären und im mobilen Einsatz ermöglicht, ohne dass der Patient von einem auf das andere Gerät umgeschaltet werden muss.

### Offenbarung der Erfindung

Diese Aufgabe wird mit der erfindungsgemäßen Beatmungsvorrichtung gemäß Anspruch 1 gelöst.

Dadurch, dass die Beatmungsvorrichtung zusätzlich zu Druckluft und Sauerstoff die Turbine zum Ansaugen von Umgebungsluft aufweist, ist zusätzlich zum herkömmlichen stationären Betrieb mit Druckluft auch ein mobiler Betrieb unter Verwendung der stets vorhandenen Umgebungsluft möglich. Dadurch deckt die Beatmungsvorrichtung alle Einsatzmöglichkeiten ab.

Die Beatmungsvorrichtung kann dazu vorgesehen sein, die Atmung des Patienten vollständig zu übernehmen; sie kann aber auch dazu vorgesehen sein, die Atmung des Patienten teilweise zu unterstützen und insbesondere ein selbstständiges Einatmen des Patienten zu ermöglichen. Der Sauerstoff aus der Sauerstoffversorgung wird in der Beatmungsvorrichtung entweder reingeführt oder mit der Luft in geeigneter Weise vermischt, sodass dem Patienten geeignete Atemluft zugeführt werden kann. Dazu weist die Beatmungsvorrichtung einen Ausgang bzw. einen Ausgangsanschluss für die Atemluft auf, an den der Patient bzw. ein Schlauchsystem des Patienten angeschlossen werden kann. Die dem Sauerstoff zugemischte Luft kann Druckluft von der Druckluftversorgung oder von der Turbine angesaugte Umgebungsluft sein.

Die Beatmungsvorrichtung kann als eigenständiges Einzelgerät verwendet werden, sie kann aber auch im Verbund mit anderen medizinischen Geräten, insbesondere in einer modulartigen Verwendung, benutzt werden. Die Sauerstoffversorgung kann eine Sauerstoffflasche und/oder eine stationäre Sauerstoffversorgung bzw. ein stationäres Sauerstoffleitungssystem zum Beispiel in einem Krankenhaus sein. Der Sauerstoff der Sauerstoffversorgung kann zum Beispiel unter einem Druck von 2,8 bar bis 6,0 bar bzw. 280 kPa bis 600 kPa vorhanden sein. Die Druckluftversorgung kann eine Druckluftflasche und/oder eine stationäre Druckluftversorgung bzw. ein stationäres Druckluftleitungssystem sein. Die Druckluft kann zum Beispiel unter einem Druck von 2,8 bar bis 6,0 bar bzw. 280 kPa bis 600 kPa vorhanden sein. Mit Turbine im Sinne dieser Anmeldung ist jegliche Ansaugeinrichtung gemeint, mit der die Umgebungsluft angesaugt werden kann. Es kann zum Beispiel vorgesehen sein, dass die Turbine einen Unterdruck von ca. 150 mbar bzw. 15 kPa erzeugt.

Der Sauerstoff fließt bzw. strömt von der Sauerstoffversorgung über den Sauerstoffeingang durch eine Sauerstoffleitung in die Beatmungsvorrichtung. Die Beatmungsvorrichtung kann einen Drucksensor aufweisen, der den Druck des Sauerstoffs in der Sauerstoffleitung misst.

Gemäß einer Ausführungsform der Erfindung weist die Beatmungsvorrichtung sowohl in der Sauerstoffleitung, beispielsweise am Eingang der Sauerstoffleitung, als auch in der Druckluftleitung, beispielsweise am Eingang der Druckluftleitung, jeweils einen Drucksensor auf. Das hat den Vorteil, dass beide Leitungen überwacht werden können, so dass ein Ausfall oder Abstecken von Druckluft und/oder Sauerstoff sofort durch die Zuschaltung der Turbine kompensiert werden kann. Es wird demnach nicht nur ausgefallene Druckluft durch die Turbine kompensiert, sondern bei Bedarf auch eine ausgefallene Sauerstoffversorgung. Dies ist insbesondere im Wechselbetrieb vorteilhaft, da das manuelle Umschalten des Patienten zwischen mobilem und stationärem Gerät beispielsweise bis zu 15 Minuten beanspruchen kann. Muss beispielsweise bei einem mobilen Einsatz, bei dem keine Druckluft zur Verfügung steht, eine Sauerstoffdruckflasche getauscht werden, so wird die Zeit, bis neuer Sauerstoff zur Verfügung steht, durch die Zuschaltung der Turbine automatisch überbrückt und die Beatmung des Patienten somit fortgesetzt. Eine angeschlossene Druckluftversorgung kann auch als Backup verwendet werden, falls die Turbine ausfällt. Für den Fall, dass keine Druckluftversorgung mittels Druckluft und Turbine möglich ist, wird mit Sauerstoff beatmet.

Die Beatmungsvorrichtung kann ein Dosierventil aufweisen, mit dem der Sauerstoff in der Sauerstoffleitung dosiert werden kann bzw. der Durchfluss des Sauerstoffs durch die Sauerstoffleitung geregelt werden kann. Des Weiteren kann die Beatmungsvorrichtung einen Durchflusssensor aufweisen, der den Durchfluss des Sauerstoffs durch die Sauerstoffleitung misst. Es kann vorgesehen sein, zusätzlich zur Beaufschlagung der Beatmungsvorrichtung über die Sauerstoffleitung auch die Turbine laufen zu lassen, um zusätzlich Umgebungsluft zuzuführen.

Wenn eine Druckluftversorgung angeschlossen ist, strömt die Druckluft über den Drucklufteingang durch eine Druckluftleitung in die Beatmungsvorrichtung. Die Beatmungsvorrichtung kann einen weiteren Drucksensor aufweisen, der den Druck der Druckluft in der Druckluftleitung misst. Die Beatmungsvorrichtung kann ein weiteres Dosierventil aufweisen, mit dem die Druckluft in der Druckluftleitung dosiert bzw. der Durchfluss der Druckluft durch die Druckluftleitung geregelt werden kann. Zusätzlich zur Druckluft kann gleichzeitig Sauerstoff über die Sauerstoffleitung zugeführt werden.

Vorteilhafte Ausgestaltungen und Weiterbildungen der Erfindung sind den Unteransprüchen, sowie der Beschreibung unter Bezugnahme auf die Zeichnungen entnehmbar.

In der Beatmungsvorrichtung werden Sauerstoff, Druckluft und/oder angesaugte Umgebungsluft miteinander vermischt. Dies kann im Leitungssystem der Beatmungsvorrichtung erfolgen.

Gemäß einer beispielhaften Weiterbildung ist jedoch vorgesehen, dass die Beatmungsvorrichtung eine Mischkammer aufweist, wobei in der Mischkammer z.B. der Sauerstoff aus der Sauerstoffversorgung mit der Druckluft aus der Druckluftversorgung oder der angesaugten Umgebungsluft vermischt wird. Insbesondere kann beim Ansaugen der Umgebungsluft mit der Turbine der Durchfluss der Luft unmittelbar durch die Turbine geregelt werden, so dass vorteilhaft auf einen Druckspeicher verzichtet werden kann, wodurch der Aufbau der Beatmungsvorrichtung wesentlich vereinfacht werden kann.

Es kann insbesondere vorgesehen sein, dass der Durchfluss der angesaugten Umgebungsluft über die Drehzahl der Turbine geregelt wird. Je nach Bedarf an Durchfluss der angesaugten Umgebungsluft kann vorteilhaft die Drehzahl der Turbine verändert werden, so dass bei hohem Bedarf die Drehzahl der Turbine erhöht und bei geringerem Bedarf die Drehzahl der Turbine verringert werden kann. Es kann insbesondere vorgesehen sein, dass der Druck, das Fließmuster und/oder das Volumen der angesaugten Luft über die Turbine einstellbar ist. Insbesondere können diese Parameter über die Drehzahl der Turbine eingestellt werden Dadurch wird der Aufbau der Beatmungsvorrichtung wesentlich vereinfacht.

In einer Ausführungsform ist es vorgesehen, dass die Beatmungsvorrichtung einen Sauerstoffeingang zum Anschluss an eine Sauerstoffversorgung aufweist, wobei über den Sauerstoffeingang Sauerstoff durch eine Sauerstoffleitung in Richtung einer Mischkammer strömen kann, und einen Drucklufteingang zum Anschluss an eine Druckluftversorgung, wobei Druckluft über den Drucklufteingang durch eine Druckluftleitung in Richtung der Mischkammer strömen kann. Die Beatmungsvorrichtung weist zusätzlich die Turbine zum Ansaugen von Umgebungsluft auf, wobei angesaugte Umgebungsluft durch eine Umgebungsluftleitung von einer Ansaugöffnung in Richtung der Mischkammer strömen kann. Die Druckluftleitung und/oder die Umgebungsluftleitung können sich mit Sauerstoff zu einer gemeinsamen Luftleitung verbinden, falls Sauerstoff vorhanden ist. Ansonsten wird nur mit Luft beatmet. Falls eine Mischkammer vorhanden ist, können die Leitungen sich vor der Mischkammer verbinden oder getrennt voneinander in die Mischkammer münden. Die Beatmungsvorrichtung weist ein Dosierventil zur Dosierung des zugeführten Sauerstoffs über den Sauerstoffeingang auf. Das Dosierventil und die Turbine sind über einen Regelkreis mit einem ersten Druck- und/oder Durchflussmesser zur Messung des Sauerstoffdrucks und/oder -durchflusses durch die Sauerstoffleitung und einem zweiten Druck- und/oder Durchflussmesser zur Messung des Luftdrucks und/oder -durchflusses durch die Luftleitung verbunden, wobei das Dosierventil und die Turbine in Abhängigkeit von dem gemessenen Sauerstoffdruck und/oder -durchfluss bzw. dem gemessenen Luftdurchfluss gesteuert werden.

Da Drucksensoren sowohl am Sauerstoffeingang als auch am Drucklufteingang vorgesehen sind, mittels derer der am Sauerstoffeingang und/oder Drucklufteingang anliegende Druck überwachbar ist, können das Dosierventil und die Turbine in Abhängigkeit von dem gemessenen Sauerstoffdruck bzw. dem gemessenen Luftdruck gesteuert werden.

Das hat den Vorteil, dass die Regelung der Beatmungsvorrichtung über die jeweiligen Durchflüsse oder die einzelnen Leitungsdrücke erfolgt und kein Druckspeicher benötigt wird. Dies ist insbesondere von Vorteil für eine mobile Einsetzbarkeit der Beatmungsvorrichtung, da ein Druckspeicher technisch aufwendig ist und zudem einen verhältnismäßig hohen Raumbedarf hat.

Es kann weiterhin vorgesehen sein, dass die Einstellungen des Durchflusses, des Drucks, des Fließmusters und/oder des Volumens der angesaugten Luft durch einen Mikroprozessor steuerbar sind, wobei die Einstellungen manuell von dem Anwender vorgegeben oder durch einen ausgewählten Beatmungsmodus automatisch vorgegeben werden können. Der Mikroprozessor erlaubt eine exakte Steuerung der oben genannten Parameter. Außerdem wird die Flexibilität bei der Verwendung der Beatmungsvorrichtung wesentlich erhöht.

Insbesondere kann die Beatmungsvorrichtung automatisch zwischen stationärem und mobilem Betrieb umschalten. Zwischen den drei Betriebsarten Druckluft, Turbine und Sauerstoff kann eine Reihenfolge bzw. Priorität automatisch festgelegt werden, es kann jedoch auch manuell umgeschaltet werden. Zu diesem Zweck sind Drucksensoren dem Sauerstoffeingang und dem Drucklufteingang zugeordnet vorgesehen, um den anliegenden Eingangsdruck für Sauerstoff und/oder Druckluft zu überwachen. Kommt es zu einem Druckabfall, etwa, weil angeschlossene Druckflaschen leer sind oder aber Versorgungsleitungen abgesteckt werden, um z.B. einen Patienten zu verlegen, wird die Turbine als Reaktion auf den registrierten Druckabfall automatisch von der Steuerung eingeschaltet, um die Beatmung aufrecht zu erhalten. Selbstverständlich kann diese Automatik abgeschaltet und die Turbine auch manuell eingeschaltet werden.

Die Beatmungsvorrichtung weist demnach Mittel auf, um festzustellen, ob Druckluft und/oder Sauerstoff angeschlossen sind bzw. ausreichend Druck aufweisen. In einer Steuerungseinheit der Beatmungsvorrichtung ist Programmcode hinterlegt, der die automatische Auswahl der Betriebsart veranlasst, wobei zur Wahl beispielsweise herangezogen werden können: Die Tatsache, ob Druckluft und/oder Sauerstoff betriebsbereit angeschlossen sind, der Bedarf an Atemluft, eine manuelle Eingabe durch einen Bediener. Beispielsweise kann es festgelegt sein, dass die Beatmung mit Druckluft und Sauerstoff erste Priorität hat, jedoch auf eine Beatmung mit angesaugter Umgebungsluft und Sauerstoff als zweite Priorität zurückgegriffen wird, falls keine Druckluft angeschlossen ist. Falls kein Sauerstoff angeschlossen ist, kann als zweite Priorität eine Beatmung ausschließlich mit Druckluft erfolgen bzw. mit dritter Priorität eine Beatmung ausschließlich mit angesaugter Umgebungsluft, wenn auch keine Druckluft angeschlossen ist.

Diese automatische Auswahl kann durch einen Bediener aufgehoben werden, indem er eine manuelle Eingabe vornimmt. Beispielsweise kann er auswählen, dass eine Beatmung ausschließlich mit Sauerstoff erfolgt, obwohl Druckluft zur Verfügung steht oder die Turbine betriebsbereit ist.

Die automatische Auswahl mit der Möglichkeit, diese manuell aufzuheben bzw. zu ändern, hat den Vorteil, dass die Beatmungsvorrichtung in jeder Situation geeignete Atemluft zur Verfügung stellt, ohne dass das Umschalten Zeit in Anspruch nimmt. Sie ist demnach besonders für den mobilen Einsatz und für den Wechsel zwischen verschiedenen Einsatzzwecken geeignet.

Es kann weiterhin beispielhaft vorgesehen sein, dass das Gasgemisch aus Luft und Sauerstoff durch eine Ausgangsleitung zum Ausgang der Beatmungsvorrichtung geleitet wird. Es kann weiterhin beispielhaft vorgesehen sein, dass die Beatmungsvorrichtung einen weiteren Durchflusssensor aufweist, der den Durchfluss an Atemluft in der Ausgangsleitung misst. Außerdem kann die Beatmungsvorrichtung einen Temperatursensor und/oder einen Drucksensor aufweisen, mit dem die Temperatur und/oder der Druck der Atemluft in der Ausgangsleitung gemessen werden kann. Dadurch ist es vorteilhaft möglich, ständig zu überprüfen, ob die gemischte Atemluft für den Patienten geeignet ist.

Wie oben beschrieben ist es vorgesehen, dass die Beatmungsvorrichtung wahlweise in einer von drei Betriebsarten (Turbine (optional mit Sauerstoff), Druckluft (optional mit Sauerstoff) und Sauerstoff)betreibbar ist, wobei die Betriebsarten von einem Anwender auswählbar sind, wobei in der ersten Betriebsart die Turbine Umgebungsluft ansaugt und keine Druckluft zugeführt wird und in der zweiten Betriebsart Druckluft von der Druckluftversorgung zugeführt wird und die Turbine ausgeschaltet ist. Dadurch ist es vorteilhaft möglich, dass der Anwender der Beatmungsvorrichtung je nach beabsichtigtem Verwendungszweck die erste oder die dritte Betriebsart auswählt, wenn ein mobiler Einsatz der Beatmungsvorrichtung gewünscht ist und zum Beispiel keine Druckluftversorgung verfügbar ist. In dieser ersten Betriebsart wird das Dosierventil in der Druckluftleitung geschlossen, so dass keine Luft durch die Druckluftleitung strömen kann. Die gesamte Luftversorgung wird in der ersten Betriebsart über die Turbine gewährleistet, so dass die Beatmungsvorrichtung unabhängig und autark von stationären Druckluftversorgungen oder Druckluftflaschen betrieben werden kann. Dadurch sind die Einsatzmöglichkeiten der Beatmungsvorrichtung erheblich erweiterbar. Die Zumischung von Sauerstoff z.B. aus einer Druckflasche ist in beiden Betriebsarten bei Bedarf möglich.

Alternativ kann die Beatmungsvorrichtung in der zweiten Betriebsart betrieben werden, wenn zum Beispiel eine stationäre Druckluftversorgung verfügbar ist. In der zweiten Betriebsart wird die Turbine ausgeschaltet, so dass vorteilhaft die Energieversorgung der Turbine geschont werden kann, wenn eine stationäre Druckluftversorgung zur Verfügung steht. Das Betreiben der Beatmungsvorrichtung in diesen beiden Betriebsarten und die variable Auswählbarkeit eine der beiden Betriebsarten je nach Verwendungszweck durch den Anwender ermöglicht eine flexible Verwendungsmöglichkeit der Beatmungsvorrichtung und erhöht das Verwendungsspektrum der Beatmungsvorrichtung in hohem Maße. Es ist allerdings auch möglich, Druckluft und Turbine parallel zu verwenden, wenn dies nötig erscheint. Dies wird steuerungsseitig realisiert, indem der Drucklufteingang, der Sauerstoffeingang und die Turbine einzeln und unabhängig voneinander schaltbar sind und zusätzlich in sinnvollen Kombinationen zusammengefasst als Betriebsprogramme abrufbar geschaltet werden können.

Die Beatmungsvorrichtung kann auch in der dritten Betriebsart verwendet werden, in der sie nur an die Sauerstoffversorgung angeschlossen ist. Es ist keine Druckluft am Drucklufteingang angeschlossen. Außerdem ist die Turbine ausgeschaltet.

Sollte am Drucksensor ein Druckabfall festgestellt werden, kann die Turbine automatisch eingeschaltet werden, um z.B. beim Abkoppeln der Druckluft zum Transport des Patienten die Beatmung aufrecht zu erhalten.

Gemäß einer anderen beispielhaften Ausführungsform sind ein Dosierventil zur Dosierung des zugeführten Sauerstoffs über den Sauerstoffeingang und die Turbine über einen Regelkreis mit einem ersten Durchflussmesser zur Messung des Sauerstoffdurchflusses und einem zweiten Durchflussmesser zur Messung des Luftdurchflusses verbunden, wobei das Dosierventil und die Turbine in Abhängigkeit von dem gemessenen Sauerstoffdurchfluss und dem gemessenen Luftdurchfluss gesteuert werden. Die Regelung erfolgt derart, dass die Durchflüsse gemessen werden und in Abhängigkeit von diesen Werten das Dosierventil und/oder die Turbine gesteuert werden, um den gewünschten Druck, das Volumen, das Fließmuster und/oder die Sauerstoffkonzentration in der Atemluft für den Patienten zu erzeugen. Der Regelkreis ermöglicht vorteilhaft eine stetige Überwachung der Durchflüsse und eine ständige Nachregelung der Turbine bzw. des Dosierventils, um stets eine geeignete Zusammensetzung der Atemluft und insbesondere eine geeignete Sauerstoffkonzentration zu erhalten.

Wie bereits erwähnt, kann die Regelung alternativ auch über die Druckmesser erfolgen statt über die Durchflussmesser.

Gemäß einer anderen beispielhaften Ausführungsform weist die Beatmungsvorrichtung ein Rückschlagventil auf, das eine Luftströmung von der Turbine in Richtung der Mischkammer ermöglicht und eine Luftströmung von der Mischkammer in Richtung der Turbine verhindert. Dadurch kann vorteilhaft erreicht werden, dass dem Patienten ein spontanes Einatmen während der Geräteexpirationsphase ermöglicht werden kann. Außerdem kann bei Betrieb in der zweiten Betriebsart ein Ausströmen der Druckluft über die Turbine verhindert werden.

Gemäß einer anderen beispielhaften Ausführungsform ist es vorgesehen, dass die Beatmungsvorrichtung eine Notentlüftungseinrichtung aufweist, die bei Überdruck in der Beatmungsvorrichtung den überschüssigen Druck, zum Beispiel aus der Ausgangsleitung, ablassen kann. Dadurch ist es vorteilhaft möglich, das Entstehen von für den Patienten gefährlichen Drücken zu verhindern, die bei der Verwendung von unter Druck stehendem Sauerstoff und/oder Druckluft entstehen können. Die Notentlüftungseinrichtung kann zum Beispiel ein in der Ausgangsleitung angeordnetes Überdruckventil aufweisen, das über ein Steuerventil geöffnet wird, so dass überschüssiger Druck aus der Ausgangsleitung entweichen kann.

Gemäß einer anderen beispielhaften Ausführungsform weist die Beatmungsvorrichtung eine derart konfigurierte Venturidüse auf, dass die Venturidüse von dem Sauerstoff aus dem Sauerstoffeingang entweder über die Hochdruckleitung (Sauerstoffflasche oder Sauerstoffzentralversorgung im Krankenhaus) oder über die Niederdruckleitung (Sauerstoffkonzentrator) durchströmt wird und dadurch einen derartigen Unterdruck erzeugt, dass Umgebungsluft angesaugt und zur Vermischung mit dem Sauerstoff zugeführt wird. Es kann insbesondere beispielhaft vorgesehen sein, dass die Beatmungsvorrichtung zusätzlich, das heißt als Ergänzung zur ersten und zweiten Betriebsart, in einer dritten Betriebsart betreibbar ist, wobei in der dritten Betriebsart die Turbine ausgeschaltet und die Druckluftzufuhr verschlossen ist, so dass die Luftversorgung ausschließlich über die von der Venturidüse angesaugte Umgebungsluft erfolgt.

Die Venturidüse kann sich in der Sauerstoffleitung befinden, falls eine Mischkammer vorhanden ist, beispielsweise vor der Mischkammer. In dieser dritten Betriebsart kann vorteilhaft auf eine Druckluftversorgung verzichtet werden und es ist kein Betrieb der Turbine erforderlich. Diese dritte Betriebsart ist daher für den mobilen Einsatz ohne stationäre Druckluftversorgung geeignet und erfordert keine Energie für die Turbine. Die dritte Betriebsart ermöglicht somit einen flexiblen und mobilen Einsatz unter Verwendung von vergleichsweise wenig Energie und erweitert die Verwendungsmöglichkeiten der Beatmungsvorrichtung erheblich. Die Zufuhr des Sauerstoffs durch die Venturidüse kann durch ein weiteres Dosierventil gesteuert werden und auch die Zufuhr der angesaugten Luft kann durch ein weiteres Dosierventil gesteuert werden. Unter anderem bestimmen die Druckverhältnisse in der Venturidüse die Menge an angesaugter Luft und damit die Sauerstoffkonzentration in der Mischkammer.

Ausführungsbeispiele der vorliegenden Erfindung sind in den Zeichnungen dargestellt und in der nachfolgenden Beschreibung näher erläutert.

### Kurze Beschreibung der Zeichnungen

### Es zeigen schematisch

- Figur 1: eine Beatmungsvorrichtung gemäß einer beispielhaften Ausführungsform der vorliegenden Erfindung,
- Figur 2: eine Beatmungsvorrichtung gemäß einer weiteren beispielhaften Ausführungsform der vorliegenden Erfindung und
- Figur 3: eine Beatmungsvorrichtung gemäß einer weiteren beispielhaften Ausführungsform der vorliegenden Erfindung.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

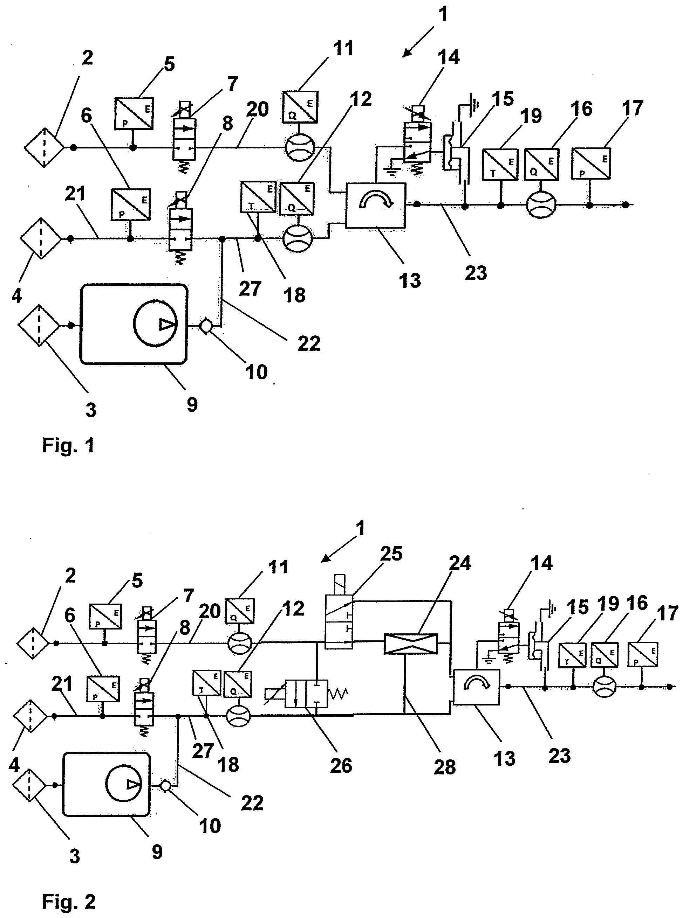

Figur 1 zeigt schematisch eine Beatmungsvorrichtung 1 gemäß einer beispielhaften Ausführungsform der vorliegenden Erfindung. Die Beatmungsvorrichtung 1 weist einen Sauerstoffeingang 2 auf. An den Sauerstoffeingang 2 wird Sauerstoff mit einem Druck von zum Beispiel 2,8 bis 6,0 bar bzw. 280 bis 600 kPa angeschlossen. Es kann auch eine Niedrigdruck-Sauerstoffzufuhr (aus dem Sauerstoff-Konzentrator) vorgesehen sein. Zwischen diesen alternativen Sauerstoff-Zuführungen kann manuell oder automatisch umgeschaltet werden. Diese Sauerstoffversorgung kann zum Beispiel durch eine Sauerstoffflasche oder eine zentrale Sauerstoffversorgung bereitgestellt werden oder durch die Sauerstoff-Niederdruckleitung aus dem Sauerstoff-Konzentrator. Der Sauerstoff strömt in einer Sauerstoffleitung 20 von dem Sauerstoffeingang 2 in Richtung einer Mischkammer 13, in die die Sauerstoffleitung 20 mündet. Die Beatmungsvorrichtung 1 weist stromabwärts vom Sauerstoffeingang 2 in der Sauerstoffleitung 20 einen Drucksensor 5 auf, der den Eingangsdruck des Sauerstoffs misst. Die Beatmungsvorrichtung 1 weist in der Sauerstoffleitung 20 ein Dosierventil 7 auf, mit dem der Sauerstoff dosiert werden kann. Die Beatmungsvorrichtung 1 weist in der Sauerstoffleitung 20 einen Durchflusssensor 11 auf, der den Durchfluss des Sauerstoffs durch die Sauerstoffleitung 20 misst.

Die Beatmungsvorrichtung 1 weist eine Ansaugöffnung 3 auf, die mit einem Filter versehen ist und zum Ansaugen von Umgebungsluft dient. Die Umgebungsluft strömt durch eine Umgebungsluftleitung 22 von der Ansaugöffnung 3 in Richtung der Mischkammer 13. Die Umgebungsluft wird durch eine Turbine 9 angesaugt. Die Turbine 9 erzeugt einen Unterdruck von ca. 150 mbar bzw. 15 kPa. In Strömungsrichtung der Umgebungsluft gesehen ist hinter der Turbine 9 ein Rückschlagventil 10 angeordnet. Das Rückschlagventil 10 öffnet nur für eine Luftströmung von der Turbine 9 in Richtung der Mischkammer 13, d.h. das Rückschlagventil 10 ist für eine Strömung von der Mischkammer 13 in Richtung der Turbine 9 verschlossen und lässt keine Strömung in dieser Richtung zu.

Die Beatmungsvorrichtung 1 weist einen Drucklufteingang 4 auf, an den Druckluft zum Beispiel mit einem Druck von 2,8 bis 6,0 bar bzw. 280 bis 600 kPa angeschlossen wird. Diese Druckluftversorgung kann zum Beispiel durch eine Druckluftflasche oder eine zentrale Druckluftversorgung bereitgestellt werden. Die Druckluft strömt durch eine Druckluftleitung 21 vom Drucklufteingang 4 in Richtung der Mischkammer 13. Die Beatmungsvorrichtung 1 weist stromabwärts vom Drucklufteingang 4 in der Druckluftleitung 21einen Drucksensor 6 auf, der den Eingangsdruck der Druckluft misst. Die Beatmungsvorrichtung 1 weist in der Druckluftleitung 21 ein Dosierventil 8 auf, mit dem die Zufuhr der Druckluft dosiert werden kann.

Die Druckluftleitung 21 verbindet sich mit der Umgebungsluftleitung 22 zu einer gemeinsamen Luftleitung 27. Die Beatmungsvorrichtung 1 weist einen Temperatursensor 18 auf, der die Temperatur der Luft in der Luftleitung 27 misst. Die Beatmungsvorrichtung 1 weist einen Durchflusssensor 12 auf, der den Durchfluss der Luft in der Luftleitung 27 misst. Die Sauerstoffleitung 20 und die Luftleitung 27 münden in die Mischkammer 13.

Das Gasgemisch verlässt die Mischkammer 13 durch eine Ausgangsleitung 23, die von der Mischkammer 13 zum Patienten bzw. zum Anschluss für das Patientenschlauchsystem führt. Die Beatmungsvorrichtung 1 weist ein Steuerventil 14 und ein Überdruckventil 15 auf. Das Steuerventil 14 dient der Steuerung des Überdruckventils 15. Die Beatmungsvorrichtung 1 weist einen Temperatursensor 19 auf, der die Temperatur des Gasgemisches in der Ausgangsleitung 23 misst, das die Mischkammer 13 verlassen hat. Die Beatmungsvorrichtung 1 weist einen Durchflusssensor 16 auf, der den Durchfluss des Gasgemisches in der Ausgangsleitung 23 misst. Die Beatmungsvorrichtung 1 weist einen Drucksensor 17 auf, der den Druck des Gasgemisches an der Patientenanschlussöffnung misst.

Die Beatmungsvorrichtung 1 kann in einer ersten Betriebsart betrieben werden, in der die Beatmungsvorrichtung 1 mit dem Sauerstoffeingang 2 an die Sauerstoffversorgung angeschlossen ist und Umgebungsluft über die Turbine 9 an der Ansaugöffnung 3 angesaugt wird. In dieser Betriebsart wird das Dosierventil 8 geschlossen, so dass die Druckluftleitung 21 verschlossen ist. Mit dem Dosierventil 7 und der Turbine 9 wird zum Beispiel mithilfe eines nicht dargestellten Mikroprozessors direkt der dem Patienten zu applizierende Druck, das Fließmuster, das Volumen und die Sauerstoffkonzentration verabreicht. Diese Parameter können entweder manuell vom Anwender eingestellt werden oder durch den gewählten Beatmungsmodus automatisch vorgegeben werden.

Um das Fließmuster und die Sauerstoffkonzentration korrekt applizieren zu können, bilden das Dosierventil 7 und die Turbine 9 zusammen mit den Durchflusssensoren 11 und 12 einen mehrstufigen Regelkreis. Die Sauerstoffkonzentration wird durch eine Sauerstoff-Gehalt-Messzelle /Sensor und/oder das Verhältnis der gemessenen Drücke und Durchflüsse der Durchflusssensoren 11 und 12 bestimmt. Der Gesamtfluss wird durch die Summe der gemessenen Drücke und Durchflüsse der Durchflusssensoren 11 und 12 bestimmt. Um den korrekten Druckverlauf anwenden zu können, bilden das Dosierventil 7 und die Turbine 9 zusammen mit dem Drucksensor 17 einen weiteren Regelkreis. Die Mischkammer 13 dient lediglich der besseren Vermischung von Sauerstoff und Luft und stellt insbesondere keinen Druckspeicher dar. Das in der Mischkammer befindliche bzw. dem Patienten zugeführte Gas wird demnach nicht auf einen konstanten, vorgegebenen Druck geregelt. Die Regelung erfolgt vielmehr über die Drucksensoren 5 und 6 und/oder über die Durchflusssensoren 11 und 12. Da die Turbine 9 auf den dynamischen Betrieb hin ausgelegt ist und somit den benötigten Fluss und Druck direkt erzeugen kann, wird kein Druckspeicher benötigt.

In einer zweiten Betriebsart der Beatmungsvorrichtung 1 wird die Luft statt aus der Umgebungsluft aus einer Druckluftflasche oder einer zentralen Luftversorgung bereitgestellt, die an den Drucklufteingang 4 angeschlossen ist. In dieser Betriebsart ist die Turbine 9 ausgeschaltet, so dass durch die Umgebungsluftleitung 22 keine Luft strömen kann. Außerdem ist das Dosierventil 8 geöffnet, so dass Druckluft durch die Druckluftleitung 21 über die Luftleitung 27 in die Mischkammer 13 strömen kann. Das Einwegventil bzw. Rückschlagventil 10 verhindert, dass der Druck über die Turbine 9 unbeabsichtigt abgebaut wird. Außerdem ermöglicht das Rückschlagventil 10, dass der Patient jederzeit während der expiratorischen Pause der Beatmungsvorrichtung einatmen kann. Der übrige Ablauf entspricht dem Ablauf in der Betriebsart mit eingeschalteter Turbine 9. Der Mikroprozessor muss die verschiedenen Eingangsdrücke dabei in den Regelkreisen bei seiner Steuerung berücksichtigen.

Es ist weiterhin mit der Beatmungsvorrichtung 1 eine Notentlüftung möglich, um das Entstehen von für den Patienten gefährlichen Drücken zu verhindern, die bei der Verwendung von unter Druck stehendem Sauerstoff und/oder Druckluft entstehen können. Zur Notentlüftung wird das Überdruckventil 15 über das Steuerventil 14 geöffnet, so dass überschüssiger Druck aus der Ausgangsleitung 23 entweichen kann.

Figur 2 zeigt schematisch eine Beatmungsvorrichtung 1 gemäß einer anderen beispielhaften Ausführungsform der vorliegenden Erfindung. Diese Ausführungsform entspricht weitgehend der Ausführungsform aus der Figur 1, wobei die Beatmungsvorrichtung 1 in dieser Ausführungsform zusätzlich zwei Dosierventile 25, 26 und eine Venturidüse 24 aufweist. Die übrigen Bestandteile entsprechen den Bestandteilen der Beatmungsvorrichtung 1 aus der Figur 1, so dass für deren Beschreibung auf die Beschreibung zur Figur 1 verwiesen werden kann.

Im Vergleich zur Beatmungsvorrichtung gemäß der Ausführungsform aus Figur 1 ist in dieser beispielhaften Ausführungsform zwischen dem Durchflusssensor 12 in der Luftleitung 27 und der Mischkammer 13 das Dosierventil 26 angeordnet. Außerdem sind in der Sauerstoffleitung 20 zwischen dem Durchflusssensor 11 und der Mischkammer 13 das Dosierventil 25 und die Venturidüse 24 angeordnet. Die Venturidüse 24 ist außerdem über eine weitere Luftleitung 28 mit der Luftleitung 27 verbunden.

In dieser beispielhaften Ausführungsform kann die Beatmungsvorrichtung 1 in einer dritten Betriebsart verwendet werden, in der die Beatmungsvorrichtung 1 nur an die Sauerstoffversorgung am Sauerstoffeingang 2 angeschlossen ist. Es ist keine Druckluft am Drucklufteingang 4 angeschlossen und das Dosierventil 8 ist geschlossen. Außerdem ist die Turbine 9 ausgeschaltet. In dieser Betriebsart ist das Dosierventil 25 derart geschaltet, dass der Sauerstoff durch die Sauerstoffleitung 20 durch die Venturidüse 24 geleitet wird. Dadurch wird in der Venturidüse 24 ein Unterdruck erzeugt, wodurch Umgebungsluft über die Luftleitung 28, die Luftleitung 27, die Umgebungsluftleitung 22, die ausgeschaltete Turbine 9 und die Ansaugöffnung 3 angesaugt wird, da das Rückschlagventil 10 eine Luftströmung von der Ansaugöffnung 3 in Richtung der Mischkammer 13 zulässt. Diese angesaugte Umgebungsluft gelangt über die Luftleitung 27 in die Mischkammer 13 und wird mit dem Sauerstoff aus der Sauerstoffleitung 20 vermischt. Die Druckverhältnisse in der Venturidüse 24 bestimmen die Menge an angesaugter Luft und damit die Sauerstoffkonzentration in der Mischkammer 13. Die Sauerstoffkonzentration kann z.B. über das Dosierventil 26 geregelt werden. Im Übrigen kann auf die Ausführungen zur Figur 1 verwiesen werden.

Sollte an einem der Drucksensoren 5, 6 ein Druckabfall festgestellt werden, kann die Turbine 9 automatisch eingeschaltet werden, um z.B. beim Abkoppeln der Druckluft zum Transport eines Patienten die Beatmung aufrechtzuerhalten.

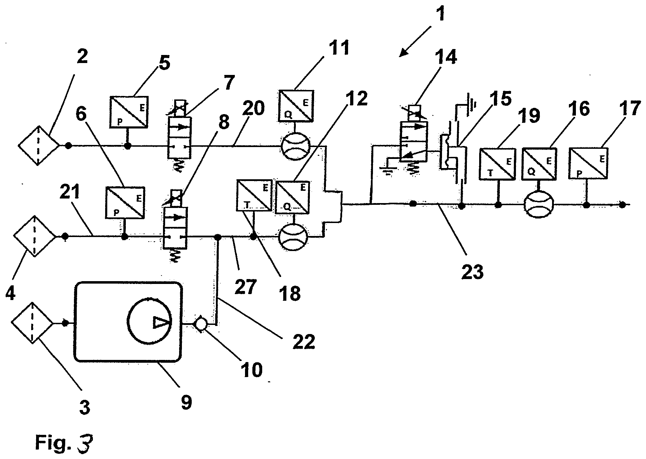

Figur 3 zeigt schematisch eine Beatmungsvorrichtung 1 gemäß einer dritten Ausführungsform der Erfindung. Diese Ausführungsform unterscheidet sich von der in Figur 1 gezeigten dadurch, dass keine Mischkammer vorgesehen ist. Stattdessen münden die Luftleitung 27 und die Sauerstoffleitung 20 in eine gemeinsame Ausgangsleitung 23, die zum Patienten bzw. zum Anschluss für das Patientenschlauchsystem führt. Die Mischung der Gase erfolgt bei dieser Ausführungsform in der Ausgangsleitung 23. Das hat den Vorteil, dass der Raumbedarf der Beatmungsvorrichtung 1 besonders gering ist, so dass sie für den mobilen Einsatz besonders geeignet ist.

Der guten Ordnung halber sei darauf hingewiesen, dass die oben beschriebenen Ausführungsbeispiele lediglich der Illustration der vorliegenden Erfindung dienen und den Gegenstand der Erfindung keinesfalls einschränken sollen.

### Bezugszeichenliste

- 1: Beatmungsvorrichtung
- 2: Sauerstoffeingang
- 3: Ansaugöffnung
- 4: Drucklufteingang
- 5, 6, 17: Drucksensor
- 7, 8: Dosierventil
- 9: Turbine
- 10: Rückschlagventil
- 11,12,16: Durchflusssensor
- 13: Mischkammer
- 14: Steuerventil
- 15: Überdruckventil
- 18, 19: Temperatursensor
- 20: Sauerstoffleitung
- 21: Druckluftleitung
- 22: Umgebungsluftleitung
- 23: Ausgangsleitung
- 24: Venturidüse
- 25, 26: Dosierventil
- 27, 28: Luftleitung

## Patentansprüche

1. Beatmungsvorrichtung (1) zum Versorgen eines Patienten mit Atemluft, aufweisend einen Sauerstoffeingang (2) zum Anschluss an eine Sauerstoffversorgung und einen Drucklufteingang (3) zum Anschluss an eine Druckluftversorgung, **wobei** die Beatmungsvorrichtung (1) zusätzlich eine Turbine (9) zum Ansaugen von Umgebungsluft aufweist, **dadurch gekennzeichnet, dass** Drucksensoren (5, 6) dem Sauerstoffeingang (2) und dem Drucklufteingang (3) zugeordnet vorgesehen sind, mittels derer der am Sauerstoffeingang (2) und/oder Drucklufteingang (3) anliegende Druck überwachbar ist und bei einem Druckabfall am Sauerstoffeingang (2) und/oder Drucklufteingang (3) die Turbine (9) automatisch einschaltbar ist.

2. Beatmungsvorrichtung (1) nach Anspruch 1, **dadurch gekennzeichnet, dass** die Beatmungsvorrichtung (1) eine Mischkammer (13) aufweist, wobei in der Mischkammer (13) der Sauerstoff aus der Sauerstoffversorgung mit der von der Turbine (9) angesaugten Umgebungsluft vermischt wird.

3. Beatmungsvorrichtung (1) nach Anspruch 1, **dadurch gekennzeichnet, dass** der Sauerstoff aus der Sauerstoffversorgung mit der von der Turbine (9) angesaugten Umgebungsluft bzw. mit Druckluft aus einer Druckluftversorgung in einer Ausgangsleitung (23) der Beatmungsvorrichtung (1) vermischt wird.

4. Beatmungsvorrichtung (1) nach einem der vorhergehenden Ansprüche, **dadurch gekennzeichnet, dass** der Durchfluss und/oder der Druck der angesaugten Umgebungsluft über die Drehzahl der Turbine (9) geregelt wird.

5. Beatmungsvorrichtung (1) nach einem der vorhergehenden Ansprüche, **dadurch gekennzeichnet, dass** die Beatmungsvorrichtung (1) in einer ersten Betriebsart oder in einer zweiten Betriebsart betreibbar ist, wobei in der ersten Betriebsart die Turbine (9) Umgebungsluft ansaugt und keine Druckluft zugeführt wird und in der zweiten Betriebsart Druckluft von der Druckluftversorgung zugeführt wird und die Turbine (9) ausgeschaltet ist.

6. Beatmungsvorrichtung (1) nach Anspruch 5, **dadurch gekennzeichnet, dass**
die Beatmungsvorrichtung (1) zusätzlich in einer dritten Betriebsart betreibbar ist, wobei in der dritten Betriebsart die Turbine (9) ausgeschaltet und die Druckluftzufuhr verschlossen ist.

7. Beatmungsvorrichtung (1) nach Anspruch 5 oder 6, **dadurch gekennzeichnet, dass** die Betriebsart automatisch nach einer vorgegebenen Priorität ausgewählt wird, wobei die automatische Auswahl durch eine manuelle Auswahl durch einen Bediener aufhebbar ist.

8. Beatmungsvorrichtung (1) nach einem der Ansprüche 5 bis 7, **dadurch gekennzeichnet, dass** in der ersten und zweiten Betriebsart zusätzlich Sauerstoff über den Sauerstoffeingang (2) zuführbar ist.

9. Beatmungsvorrichtung (1) nach einem der vorhergehenden Ansprüche, **dadurch gekennzeichnet, dass** die Einstellungen des Drucks, des Fließmusters und/oder des Volumens der von der Turbine (9) angesaugten Luft durch einen Mikroprozessor steuerbar sind, wobei die Einstellungen manuell von einem Anwender vorgenommen oder durch einen ausgewählten Beatmungsmodus automatisch vorgegeben werden können.

10. Beatmungsvorrichtung (1) nach einem der vorhergehenden Ansprüche, **dadurch gekennzeichnet, dass** die Beatmungsvorrichtung (1) ein Dosierventil (7) zur Dosierung des zugeführten Sauerstoffs über den Sauerstoffeingang (2) aufweist, wobei das Dosierventil (7) und die Turbine (9) über einen Regelkreis mit einem ersten Durchflussmesser (11) zur Messung des Sauerstoffdurchflusses und einem zweiten Durchflussmesser (12) zur Messung des Luftdurchflusses verbunden sind, wobei das Dosierventil (7) und die Turbine (9) in Abhängigkeit von dem gemessenen Sauerstoffdurchfluss und/oder dem gemessenen Luftdurchfluss steuerbar sind.

11. Beatmungsvorrichtung (1) nach einem der Ansprüche 1 bis 9, **dadurch gekennzeichnet, dass** die Beatmungsvorrichtung (1) ein Dosierventil (7) zur Dosierung des zugeführten Sauerstoffs über den Sauerstoffeingang (2) aufweist, wobei das Dosierventil (7) und die Turbine (9) über einen Regelkreis mit einem ersten Drucksensor (5) zur Messung des Sauerstoffdrucks und einem zweiten Drucksensor (6) zur Messung des Luftdrucks verbunden sind, wobei das Dosierventil (7) und die Turbine (9) in Abhängigkeit von dem gemessenen Sauerstoffdruck und/oder dem gemessenen Luftdruck steuerbar sind.

12. Beatmungsvorrichtung (1) nach einem der vorhergehenden Ansprüche, **dadurch gekennzeichnet, dass** die Beatmungsvorrichtung (1) ein Rückschlagventil (10) aufweist, das eine Luftströmung von der Turbine (9) in Richtung des Patienten und eine Spontanatmung während der Expirationsphase ermöglicht und eine Luftströmung in Richtung der Turbine (9) verhindert.

13. Beatmungsvorrichtung (1) nach einem der vorhergehenden Ansprüche, **dadurch gekennzeichnet, dass** die Beatmungsvorrichtung (1) eine Notentlüftungseinrichtung (14, 15) aufweist, die derart konfiguriert ist, dass bei Überdruck in der Beatmungsvorrichtung (1) der überschüssige Druck abgelassen werden kann.

14. Beatmungsvorrichtung (1) nach einem der vorhergehenden Ansprüche, **dadurch gekennzeichnet, dass** die Beatmungsvorrichtung (1) eine derart konfigurierte Venturidüse (24) aufweist, dass die Venturidüse (24) von dem Sauerstoff aus dem Sauerstoffeingang (2) durchströmt wird und dadurch einen derartigen Unterdruck erzeugt, dass Umgebungsluft angesaugt und zur Vermischung mit dem Sauerstoff zugeführt wird.

15. Beatmungsvorrichtung (1) nach Anspruch 14, **dadurch gekennzeichnet, dass** in der dritten Betriebsart eine Luftversorgung ausschließlich über die von der Venturidüse (24) angesaugte Umgebungsluft erfolgt.

## Claims

1. Respiratory device (1) for supplying breathing air to a patient, comprising an oxygen inlet (2) to be connected to an oxygen supply and a compressed air inlet (3) to be connected to a compressed air supply, **wherein** the respiratory device (1) additionally comprises a turbine (9) for sucking in ambient air, **characterised in that** pressure sensors (5, 6) assigned to the oxygen inlet (2) and the compressed air inlet (3) are provided, by means of which the pressure applied to the oxygen inlet (2) and/or the compressed air inlet (3) can be monitored and when there is a pressure drop at the oxygen inlet (2) and/or the compressed air inlet (3) the turbine (9) can be automatically turned on.

2. Respiratory device (1) according to claim 1, **characterised in that** the respiratory device (1) has a mixing chamber (13), wherein the oxygen from the oxygen supply is mixed in the mixing chamber (13) with the ambient air sucked in by the turbine (9).

3. Respiratory device (1) according to claim 1, **characterised in that** the oxygen from the oxygen supply is mixed with the ambient air sucked in by the turbine (9) or with compressed air from the compressed air supply in an outlet line (23) of the respiratory device (1).

4. Respiratory device (1) according to any one of the previous claims **characterised in that** the through-flow and/or the pressure of the sucked-in ambient air is regulated via the rotational speed of the turbine (9).

5. Respiratory device (1) according to any one of the previous claims, **characterised in that** the respiratory device (1) can be operated in a first mode of operation or in a second mode of operation, wherein in the first mode of operating the turbine (9) sucks in ambient air and no compressed air is supplied and in the second mode of operation compressed air is supplied from the compressed air supply and the turbine (9) is turned off.

6. Respiratory device (1) according to claim 5, **characterised in that** the respiratory device (1) can additionally be operated in a third mode of operation, wherein in the third mode of operation the turbine (9) is turned off and the compressed air supply is closed.

7. Respiratory device (1) according to claim 5 or 6, **characterised in that** the mode of operation is automatically selected according to a pre-determined priority, wherein the automatic selection can be cancelled by a manual selection by an operator.

8. Respiratory device (1) according to any one of claims 5 to 7 **characterised in that** in addition oxygen can be supplied via the oxygen inlet (2) in the first and second mode of operation.

9. Respiratory device (1) according to any one of the previous claims, **characterised in that** the settings of the pressure, the flow pattern and/or the volume of the air sucked in by the turbine (9) can be controlled by a microprocessor, wherein the settings can be made manually by a user or can be automatically predetermined by a selected ventilation mode.

10. Respiratory device (1) according to any one of the previous claims, **characterised in that** the respiratory device (1) has a metering valve (7) for metering the oxygen supplied via the oxygen inlet (2), wherein the metering valve (7) and the turbine (9) are connected via a control circuit to a first through-flow measuring device (11) for measuring the oxygen through-flow and a second through-flow measuring device (12) for measuring the air through-flow, wherein the metering valve (7) and the turbine (9) can be controlled in dependence on the measured oxygen through-flow and/or the measured air through-flow.

11. Respiratory device (1) according to any one of claims 1 to 9, **characterised in that** the respiratory device (1) has a metering valve (7) for metering the oxygen supplied via the oxygen inlet (2), wherein the metering valve (7) and the turbine (9) are connected via a control circuit to a first pressure sensor (5) for measuring the oxygen pressure and a second pressure sensor (6) for measuring the air pressure, wherein the metering valve (7) and the turbine (9) can be controlled in dependence on the measured oxygen pressure and/or the measured air pressure.

12. Respiratory device (1) according to any one of the previous claims **characterised in that** the respiratory device (1) has a check valve (10), which makes possible an air flow from the turbine (9) in the direction of the patient and a spontaneous breathing during the expiration phase and prevents an air flow in the direction of the turbine (9).

13. Respiratory device (1) according to any one of the previous claims, **characterised in that** the respiratory device (1) has an emergency venting device (14, 15), which is configured in such a manner that in the event of excess pressure in the respiratory device (1) the excess pressure can be released.

14. Respiratory device (1) according to any one of the previous claims, **characterised in that** the respiratory device (1) has a venture nozzle (24) configured in such a manner that the venture nozzle (24) is flowed through by the oxygen from the oxygen inlet (2) and thus generates such a negative pressure that ambient air is sucked in and is supplied to be mixed with the oxygen.

15. Respiratory device (1) according to claim 14, **characterised in that** in the third mode of operation an air supply is provided exclusively via the ambient air sucked in by the venture nozzle (24).

## Revendications

1. Respirateur (1) destiné à alimenter un patient en air à respirer, comportant une entrée d'oxygène (2) destinée à être raccordée à une alimentation en oxygène et une entrée d'air comprimé (3) destinée à être raccordée à une alimentation en air comprimé, dans lequel le respirateur (1) comporte en plus une turbine (9) destinée à aspirer de l'air ambiant, **caractérisé en ce que** des capteurs de pression (5, 6) sont associés à l'entrée d'oxygène (2) et à l'entrée d'air comprimé (3), capteurs de pression au moyen desquels la pression existant au niveau de l'entrée d'oxygène (2) et/ou de l'entrée d'air comprimé (3) peut être surveillée et la turbine (9) peut être enclenchée automatiquement lors d'une chute de pression au niveau de l'entrée d'oxygène (2) et/ou de l'entrée d'air comprimé (3).

2. Respirateur (1) selon la revendication 1, **caractérisé en ce que** le respirateur (1) comporte une chambre de mélange (13), l'oxygène issu de l'alimentation en oxygène étant mélangé dans la chambre de mélange (13) avec l'air ambiant aspiré par la turbine (9).

3. Respirateur (1) selon la revendication 1, **caractérisé en ce que** l'oxygène issu de l'alimentation en oxygène est mélangé avec l'air ambiant aspiré par la turbine (9) ou avec de l'air comprimé issu d'une alimentation en air comprimé dans une conduite de sortie (23) du respirateur (1).

4. Respirateur (1) selon l'une quelconque des revendications précédentes, **caractérisé en ce que** le débit et/ou la pression de l'air ambiant aspiré est réglé par l'intermédiaire de la vitesse de rotation de la turbine (9).

5. Respirateur (1) selon l'une quelconque des revendications précédentes, **caractérisé en ce que** le respirateur (1) peut fonctionner dans un premier mode de fonctionnement ou dans un deuxième mode de fonctionnement et, dans le premier mode de fonctionnement, la turbine (9) aspire de l'air ambiant et aucun air comprimé n'est amené et, dans le deuxième mode de fonctionnement, de l'air comprimé est amené par l'alimentation en air comprimé et la turbine (9) est arrêtée.

6. Respirateur (1) selon la revendication 5, **caractérisé en ce que** le respirateur (1) peut fonctionner en plus dans un troisième mode de fonctionnement et, dans le troisième mode de fonctionnement, la turbine (9) est arrêtée et l'amenée d'air comprimé est fermée.

7. Respirateur (1) selon la revendication 5 ou 6, **caractérisé en ce que** le mode de fonctionnement est sélectionné automatiquement selon une priorité prédéterminée, la sélection automatique pouvant être annulée par une sélection manuelle effectuée par un opérateur.

8. Respirateur (1) selon l'une quelconque des revendications 5 à 7, **caractérisé en ce que**, dans le premier et dans le deuxième mode de fonctionnement, de l'oxygène peut être amené en plus par l'intermédiaire de l'entrée d'oxygène (2).

9. Respirateur (1) selon l'une quelconque des revendications précédentes, **caractérisé en ce que** les réglages de la pression, de la configuration d'écoulement et/ou du volume de l'air aspiré par la turbine (9) peuvent être commandés par un microprocesseur, les réglages pouvant être effectués manuellement par un utilisateur ou être prescrits automatiquement par un mode de respiration sélectionné.

10. Respirateur (1) selon l'une quelconque des revendications précédentes, **caractérisé en ce que** le respirateur (1) comporte une vanne de dosage (7) destinée à doser l'oxygène amené par l'intermédiaire de l'entrée d'oxygène (2), laquelle vanne de dosage (7) et la turbine (9) sont reliées par l'intermédiaire d'un circuit de régulation à un premier débitmètre (11) destiné à mesurer le débit d'oxygène et à un deuxième débitmètre (12) destiné à mesurer le débit d'air, laquelle vanne de dosage (7) et la turbine (9) peuvent être commandées en fonction du débit d'oxygène mesuré et du débit d'air mesuré.

11. Respirateur (1) selon l'une quelconque des revendications 1 à 9, **caractérisé en ce que** le respirateur (1) comporte une vanne de dosage (7) destinée à doser l'oxygène amené par l'intermédiaire de l'entrée d'oxygène (2), laquelle vanne de dosage (7) et la turbine (9) sont reliées par l'intermédiaire d'un circuit de régulation à un premier capteur de pression (5) destiné à mesurer la pression d'oxygène et à un deuxième capteur de pression (6) destiné à mesurer la pression d'air, laquelle vanne de dosage (7) et la turbine (9) peuvent être commandées en fonction de la pression d'oxygène mesurée et de la pression d'air mesurée.

12. Respirateur (1) selon l'une quelconque des revendications précédentes, **caractérisé en ce que** le respirateur (1) comporte un clapet antiretour (10) qui permet un écoulement d'air de la turbine (9) en direction du patient et une respiration spontanée pendant la phase d'expiration et empêche un écoulement d'air en direction de la turbine (9).

13. Respirateur (1) selon l'une quelconque des revendications précédentes, **caractérisé en ce que** le respirateur (1) comporte un dispositif de purge d'air de secours (14, 15) qui est configuré de telle sorte que, en cas de surpression dans le respirateur (1), la pression excédentaire peut être abaissée.

14. Respirateur (1) selon l'une quelconque des revendications précédentes, **caractérisé en ce que** le respirateur (1) comporte un tube de Venturi (24) configuré de telle sorte que le tube de Venturi (24) est traversé par l'oxygène issu de l'entrée d'oxygène (2) et produit ainsi une dépression telle que de l'air ambiant est aspiré et est amené pour le mélange avec l'oxygène.

15. Respirateur (1) selon la revendication 14, **caractérisé en ce que**, dans le troisième mode de fonctionnement, une alimentation en air s'effectue exclusivement par l'intermédiaire de l'air ambiant aspiré par le tube de Venturi (24).
